(19) Europäisches Patentamt
European Patent Office
Office européen des brevets

(11) Veröffentlichungsnummer: **0 259 678 A2**

# (12) EUROPÄISCHE PATENTANMELDUNG

(21) Anmeldenummer: **87112187.7**

(51) Int. Cl.4: **A61K 7/075**

(22) Anmeldetag: **21.08.87**

(30) Priorität: **29.08.86 DE 3629510**

(43) Veröffentlichungstag der Anmeldung: **16.03.88 Patentblatt 88/11**

(84) Benannte Vertragsstaaten: **AT BE CH DE ES FR GB IT LI NL SE**

(71) Anmelder: **Henkel Kommanditgesellschaft auf Aktien**
**Postfach 1100 Henkelstrasse 67**
**D-4000 Düsseldorf-Holthausen(DE)**

(72) Erfinder: **Höffkes, Horst, Dr.**
**Carlo-Schmid-Strasse 113**
**D-4000 Düsseldorf(DE)**
Erfinder: **Lange, Fritz, Dr.**
**Baderweg 82**
**D-4300 Essen(DE)**
Erfinder: **Giede, Karl**
**Schlehenweg 12**
**D-4010 Hilden(DE)**

## (54) Mittel zur Pflege und Nachbehandlung der Haare.

(57) Mittel zur Pflege und Nachbehandlung von Haaren enthalten oberflächenaktive quartäre Ammoniumverbindungen der Formel

$$R^1 - (OC_nH_{2n})_x - \overset{R^3}{\underset{(C_nH_{2n}O)_zH}{\overset{(+)}{N}}} - (C_nH_{2n}O)_y - R^2 \qquad A^{(-)}$$

als Avivagewirkstoffe mit besonders gutem Emulgiervermögen. Bevorzugt liegen diese Mittel als Öl-in-Wasser-Emulsion vor und enthalten 0,1 bis 5 Gew.-% der quartären Ammoniumverbindung und 0,2 bis 10 Gew.-% wasserunlösliche Fettkomponenten aus der Gruppe Fettalkohole mit 12 bis 18 C-Atomen, Fettsäuremono-und/oder -diglyceride von Fettsäuren mit 12 bis 18 C-Atomen oder Paraffine.

EP 0 259 678 A2

# Mittel zur Pflege und Nachbehandlung der Haare

Gegenstand der Erfindung sind kosmetische Mittel zur Pflege und Nachbehandlung der Haare mit einem Gehalt an oberflächenaktiven quartären Ammoniumverbindungen mit zwei Fettalkyloxyalkylgruppen. Hierunter werden Zusammensetzungen verstanden, die nach der Haarwäsche auf das Haar aufgebracht werden, um dessen Kämmbarkeit, Glanz und Fülle zu verbessern und dessen statische Aufladbarkeit zu verringern und die dann wieder aus dem Haar ausgespült werden sowie solche Zusammensetzungen, die auf das Haar aufgebracht werden, um dessen Struktur zu verbessern oder eine Festigung, Wasserwelle, Dauerwellung, Färbung oder Bleichung zu erzielen. In solchen Zubereitungen werden seit langem quartäre Ammoniumverbindungen wegen deren Substantivität und antistatischer und avivierender Eigenschaften eingesetzt. Während die quartären Ammoniumverbindungen mit einer langen Fettalkylgruppe am quartären Stickstoffatom gute antistatische Eigenschaften, aber eine geringe Avivagewirkung aufweisen, zeigen solche mit zwei langen Fettalkylgruppen am quartären Stickstoffatom eine bessere Avivagewirkung. Die Verbindungen weisen jedoch nur ein begrenztes Emulgiervermögen auf. Daher müssen in emulsionsförmigen Zusammensetzungen zusätzliche nichtionogene Emulgatoren eingesetzt werden.

Es wurde gefunden, daß quartäre Ammoniumverbindungen mit zwei Fettalkyl-oxyalkylgruppen sowohl eine noch weiter verbesserte Avivagewirkung als auch ein besonders gutes Emulgiervermögen für kosmetische Öl-und Fettkomponenten aufweisen. Gegenstand der Erfindung sind daher Mittel zur Pflege und Nachbehandlung von Haaren mit einem Gehalt an oberflächenaktiven quartären Ammoniumverbindungen, die als quartäre Ammoniumverbindungen solche der Formel I

$$\text{I} \qquad R^1-(OC_nH_{2n})_x-\overset{\overset{\displaystyle R^3}{|}}{\underset{\underset{\displaystyle (C_nH_{2n}O)_zH}{|}}{\overset{(+)}{N}}}-(C_nH_{2n}O)_y-R^2 \qquad A^{(-)}$$

enthalten, in welcher $R^1$ und $R^2$ lineare Alkylgruppen mit 8 bis 22 C-Atomen, $R^3$ eine Alkylgruppe mit 1 bis 4 C-Atomen, eine Benzylgruppe oder eine Gruppe $-(C_nH_{2n}O)_zH$, $n = 2$ oder 3, x, y und z Zahlen von 1 bis 20 und A ein Chlorid, Bromid oder ein Anion der Formel $R^4OSO_3^{(-)}$ darstellt, worin $R^4$ eine Alkylgruppe mit 1 bis 4 C-Atomen ist.

Bevorzugt enthalten die erfindungsgemäßen Zubereitungen solche quartären Ammoniumverbindungen der Formel I, in welchen $R^1$ und $R^2$ lineare Alkylgruppen mit 16 bis 18 C-Atomen und $R^3$ eine Methylgruppe oder Hydroxyethylgruppe ist.

Die quartären Ammoniumverbindungen der Formel I lassen sich aus tertiären Etheraminen der Formel II

$$\text{II} \qquad R^1-(OC_nH_{2n})_x-\underset{\underset{\displaystyle (C_nH_{2n}O)_zH}{|}}{N}-(C_nH_{2n}O)_y-R^2$$

durch Quaternierung mit Alkylhalogeniden mit 1 bis 4 C-Atomen, Dialkylsulfaten mit Alkylgruppen mit 1 bis 4 C-Atomen, mit Benzylhalogeniden oder mit Ethylenoxid oder aus tertiären Etheraminen der Formel III

$$\text{III} \qquad R^1-(OC_nH_{2n})_x-\overset{\overset{\displaystyle R^3}{|}}{N}-(C_nH_{2n}O)_y-R^2$$

durch Quaternierung mit Ethylenoxid herstellen. Die Quaternierung der Etheramine der Formel II mit Ethylenoxid nach bekanntem Verfahren führt zu einem quartären Ammoniumhydroxid der Formel I, in welchem $R^3$ eine Gruppe $-(C_nH_{2n}O)_zH$ darstellt. Dieses muß dann durch Neutralisation z. B. mit einer

Mineralsäure oder einer wasserlöslichen Carbonsäure mit 1 bis 4 C-Atomen in die Salzform überführt werden. In solchen Fällen kann $A^{(-)}$ auch das Anion einer anderen Mineralsäure, z. B. ein Sulfat-oder Phosphat-Anion oder einer niederen Carbonsäure, z. B. ein Acetat-, Glycolat-, Lactat-, Citrat-oder Tartrat-Anion sein.

Die tertiären Etheramine der Formel III sind ausgehend von tertiären Etheraminen der Formel IV

$$\text{IV} \quad R^1 - (OC_nH_{2n})_x - \underset{\displaystyle\phantom{x}}{\overset{\displaystyle R^3}{\overset{|}{N}}} - (C_nH_{2n}O)_y - H$$

durch Alkylierung an der Hydroxylgruppe mit Hilfe von Schwefelsäurehalbestern oder Schwefelsäurehalbestersalzen von Fettalkoholen mit 8 bis 22 C-Atomen zugänglich. Ein besonders elegantes Verfahren zur Herstellung tertiärer Etheramine ist in der deutschen Patentanmeldung P 35 04 242.7 beschrieben. Die Herstellung von zwei bisher nicht bekannten quartären Ammoniumverbindungen der Formel I wird als Beispiel beschrieben. Quartäre Ammoniumverbindungen der Formel I, in welcher y und ggf. z Werte von mehr als 1 aufweisen, sind ausgehend von tertiären Etheraminen der Formel IV, in welchen y und ggf. z den Wert 1 aufweisen, durch Anlagerung von (y - 1) + (z - 1) Mol Ethylenoxid nach bekanntem Verfahren, Alkylierung zur Verbindung der Formel II und Quaternierung wie beschrieben herstellbar.

In den Formeln II, III und IV haben $R^1$, $R^2$, $R^3$, n, x, y und z die gleiche Bedeutung wie für Formel I angegeben.

Eine bevorzugte Ausführung der Erfindung sind Mittel zur Nachbehandlung der Haare nach dem Waschen, sogenannte Haarspülmittel, in Form einer Öl-in-Wasser-Emulsion, die 0,1 bis 5 Gew.-% der quartären Ammoniumverbindung der Formel I und zusätzlich eine wasserunlösliche Öl-und/oder Fettkomponente, bevorzugt aus der Gruppe der Fettalkohole mit 12 bis 18 C-Atomen und der Fettsäuremono-und/oder diglyceride von Fettsäuren mit 12 bis 18 C-Atomen und der Paraffine enthalten. Weitere geeignete Öl und/oder Fettkomponenten sind kosmetische Öle, z. B. 2-Octyldodecanol, Fettsäure $(C_{8/10})$-triglyceride, Oleylerucat, Decyloleat, Isopropylstearat, Squalan und Fett-oder Wachskomponenten wie z. B. Lanolin, Walrat, Japanwachs, Bienenwachs oder synthetische Ersatzprodukte für diese Naturstoffe. Die kosmetischen Öl-oder Fettkomponenten sind bevorzugt in einer Menge von 0,2 bis 10 Gew.-% in solchen emulsionsförmigen Zubereitungen enthalten. Darüber hinaus können solche Zubereitungen weitere übliche Bestandteile, z. B. nichtionogene Tenside, kationische Tenside, z. B. quartäre Ammoniumverbindungen mit einer Fettalkylgruppe an Stickstoffatom, wasserlösliche kationische Polymere, wasserlösliche nichtionische Polymere, nichtionische Emulgatoren, Propylenglycol, Glycerin, Glucose, Kräuterextrakte, Vitamine, Sebostatika, Antischuppenwirkstoffe, Konservierungsmittel, Farbstoffe, Duftstoffe und Trübungs-und Perlglanzmittel in den üblichen Mengen enthalten.

Eine weitere bevorzugte Ausführungsform sind Mittel zur Pflege der Haare in Form einer klaren, wäßrigen oder wäßrig-alkoholischen flüssigen oder gelförmigen Zubereitung, die 0,1 bis 5 Gew.-% der quartären Ammoniumverbindung der Formel I und zusätzlich ein verdickendes, festigendes oder konditionierendes wasserlösliches Polymeres in einer Menge von 0,1 bis 5 Gew.-% enthält. Als wasserlösliche Polymere können anionische, kationische oder nichtionische wasserlösliche Polymere mit einem mittleren Molekulargewicht von 1 000 bis 1 000 000 verwendet werden. Ge eignete anionische Polymere sind z. B. Polymerisate von Acrylsäure, Methacrylsäure, Crotonsäure, Itaconsäure und Maleinsäure sowie deren Copolymerisate mit einem monoäthylenisch ungesättigten Monomeren, insbesondere Ethylen, Vinylbenzol, Vinylacetat, Vinylmethylether oder Acrylamid. Andere geeignete anionische Polymere sind die aus DE-A 24 52 031 bekannten und unter der Handelsbezeichnung POC (Degussa) erhältlichen Carboxyl-bzw. Carboxylat-und Aldehydgruppen aufweisenden Polymeren oder die aus US-PS 2,798,053 bekannten und unter der Handelsbezeichnung Carbopol ® erhältlichen vernetzten Acrylsäurepolymerisate. Schließlich können auch wasserlösliche Carboxymethylcellulosen oder Carboxymethylstärke eingesetzt werden.

Geeignete kationische Polymere sind z. B. die aus US-PS 3,912,808 bekannten und unter der Handelsbezeichnung Merquat ® 100 oder Merquat ® 500 (Quaternium 41) erhältlichen quaternierten Polymeren und Copolymeren des Dimethyldiallylammoniumchlorids, die aus US-PS 3,472,840 bekannten und unter der Handelsbezeichnung Polymer JR ® 400 erhältlichen kationischen Celluloseether, die aus US-PS 3,910,862 bekannten und unter der Handelsbezeichnung Gafquat ® 734 und 755 erhältlichen kationischen Polyvinylpyrrolidon-Copolymerisate, die aus US-PS 3,632,559 bekannten und unter der Handelsbezeichnung Cartaretine ® F4 erhältlichen Copolymeren aus Adipinsäure und Dimethylaminohydroxypropyl-diethylentriamin und die aus US-PS 4,157,388 bekannten und unter der Handelsbezeichnung Mirapol ® A15 erhältlichen quartären polymeren Harnstoffderivate.

Geeignete nichtionische Polymere sind z. B. Polyvinylpyrrolidon, die unter der Handelsbezeichnung Luviskol ® oder Kollidon ® erhältlich sind, oder Hydroxyethylcellulosen, Hydroxypropylcellulosen oder Methylhydroxypropylcellulose. Darüber hinaus können diese klaren, flüssigen oder gelförmigen Zubereitungen weitere übliche Bestandteile, z. B. nichtionogene Tenside bzw. Emulgatoren, Propylenglycol, Glycerin, Glucose, Kräuterextrakte, Vitamine, Sebostatika, Antischuppenwirkstoffe, Konservierungsmittel, Farbstoffe und Duftstoffe in den üblichen Mengen enthalten.

Die folgenden Beispiele sollen den Erfindungsgegenstand näher erläutern, ohne ihn hierauf zu beschränken:

## Beispiele

1. Herstellung von Cetyl-/Stearylpoly(6)oxyethyl-Cetyl-/Stearyloxyethyl-hydroxyethyl-methyl-ammoniumchlorid

1.1 Cetyl-/Stearylpoly(6)oxyethyl-dihydroxyethyl-amin

1635 g Cetyl-/Stearyl (30:70) poly(5)oxyethyl-schwefelsäurehalbester
528,2 g Triethanolamin und
566,4 g Natriumhydroxidlösung (50 Gew.-% in Wasser)

wurden unter Kühlung gemischt und das Gemisch auf 175 °C erwärmt, wobei ab 100 °C Wasser unter vermindertem Druck abdestilliert wurde. Nach 2 Stunden Reaktionszeit bei 200 °C wurde auf 80 °C abgekühlt, mit gesättigter Kochsalzlösung gewaschen und dann im Vakuum am Reaktionsverdampfer bei 80 °C getrocknet. Es wurden 1460 g eines dunklen Öles mit einer Aminzahl von 69,3 und einer Hydroxylzahl von 140,6 erhalten.

1.2 Cetyl-/Stearylpoly(6)oxyethyl-Cetyl-/Stearyloxyethylhydroxyethylamin

300 g (0,375 Mol) des Reaktionsproduktes aus 2.1 wurden auf 80 °C erwärmt. Dann wurden portionsweise 258,8 g (0,375 Mol) Cetyl/Stearyl-sulfat-Natriumsalz (55%ige Paste) zugesetzt und unter Vakuum das Wasser abdestilliert. Schließlich wurden 18,0 g (0,45 Mol) Natriumhydroxid zugesetzt und der Ansatz auf 175 °C erhitzt. Nach 2 Stunden Reaktionszeit wurde auf 80 °C gekühlt und zweimal mit gesättigter Kochsalzlösung gewaschen und das Reaktionsprodukt dann im Vakuum am Reaktionsverdampfer getrocknet.

1.3 Quaternierung

187,5 g des Reaktionsproduktes aus 2.2 wurden in 40 g Isopropanol und 22,5 g Wasser gelöst und mit 100 g Methylchlorid im Druckautoklaven bei 90 °C und 10 bar zur Umsetzung gebracht. Nach ca. 4 Stunden war die Umsetzung beendet. Nach Entspannung wurden Wasser und Isopropanol am Reaktionsverdampfer bei 80 °C entfernt.

2. Herstellung von Di-(Cetyl-/Stearyloxyethyl)-hydroxyethylmethyl-ammoniumchlorid

2.1 Cetyl-/Stearyloxyethyl-dihydroxyethylamin 21,0 kg (30,4 Mol) Cetyl-/Stearylsulfat-Na-Salz (55%ige Paste)
10,7 kg (72 Mol) Triethanolamin
1,74 kg (43 Mol) Natriumhydroxid

Das Triethanolamin wurde im Kessel vorgelegt und das Cetyl-/Stearylsulfat-Na-Salz portionsweise zugesetzt und bei vermindertem Druck entwässert. Dann wurde das Natriumhydroxid zugefügt. Nach 2 Stunden Reaktionszeit bei 175 °C wurde auf 80 °C abgekühlt, zweimal mit entsalztem Wasser gewaschen und dann im Vakuum bei 110 °C getrocknet. Es wurden 12,5 kg eines gelblichen festen Produkts mit einer Aminzahl von 135 erhalten.

2.2 Di-(Cetyl-/Stearyloxyethyl)-hydroxyethyl-amin

8 kg (19,8 Mol) des Reaktionsproduktes nach 3.1 wurden auf 90 °C erwärmt. Dann wurden portionsweise 13,4 kg (19,8 Mol) Cetyl-/Stearylsulfat-Na-Salz (55%ige Paste) zugesetzt und unter Vakuum das Wasser abdestilliert. Schließlich wurden noch 930 g Natriumhydroxid zugesetzt und der Ansatz auf 200 °C erhitzt. Nach 3 Stunden Reaktionszeit wurde zweimal mit entsalztem Wasser bei 95 °C gewaschen und das Reaktionsprodukt im Vakuum bei 110 °C getrocknet. Es wurden 12 kg eines hellgelben festen Produkts mit einer Aminzahl von 84 erhalten.

2.3 Quaternierung

500 g des Reaktionsprodukts nach 3.2 wurden in 107 g Isopropanol und 60 g Wasser gelöst und mit 200 g Methylchlorid im Druckautoklaven bei 90 °C und 5 bar zur Umsetzung gebracht. Nach etwa 2,5 Stunden war die Umsetzung beendet. Nach Entspannung wurden Wasser und Isopropanol am Rotationsverdampfer bei 80 °C entfernt.

3. Herstellung von Di-(Cetyl-/Stearyloxyethyl)-di(poly(3)oxyethyl)-ammoniumchlorid

224,5 g (0,31 Mol) des Reaktionsprodukts nach 3.2, 31,0 g (0,31 Mol) Milchsäure (90%ig) und 76,9 g Wasser wurden in einem Druckautoklaven vorgelegt. Nach Spülung mit Stickstoff wurden bei einer Temperatur von 80 °C mit einem Maximaldruck von 5 bar 67,8 g (1,54 Mol) Ethylenoxid aufgedrückt. Nach etwa 4 Stunden Nachreaktionszeit bei 85 °C und anschließender Entspannung wurden 396 g einer bei Raumtemperatur festen Masse mit einer Säurezahl von 0,7 erhalten.

<u>Anwendungsbeispiele</u>

4.1 Haarnachspülmittel Produkt gemäß Beispiel 1    1 Gew.-%
Cetyl/Stearylalkohol (50:50)    1,5 Gew.-%
Wasser    ad 100 Gew.-%

4.2 Fönwell-Lotion Ethanol    30 Gew.-%
Gafquat ® 755 (1)    1 Gew.-%
Luviskol ® K 30 (2)    0,5 Gew.-%
Cremophor RH 30 (3)    0,3 Gew.-%
Produkt gemäß Beispiel 2.3    0,15 Gew.-%
Wasser    ad 100 Gew.-%

Es wurden folgende Handelsprodukte verwendet:

(1) Gafquat 755: Copolymer aus Methacrylsäuredimethylaminoethylester und Vinylpyrrolidon, quaterniert mit Dimethylsulfat, entsprechend US-PS 3,910,862 (Hersteller GAF-Corporation)

(2) Luviskol K 30: Polyvinylpyrrolidon, K-Wert: ca. 30 (Hersteller: BASF-AG)

(3) Cremophor RH 30: Hydriertes Ricinusöl + 60 Mol Ethylenoxid (Hersteller: BASF-AG)

## Ansprüche

1. Mittel zur Pflege und Nachbehandlung von Haaren mit einem Gehalt an oberflächenaktiven quartären Ammoniumverbindungen, dadurch gekennzeichnet, daß als quartäre Ammoniumverbindungen solche der Formel 1

$$\text{I} \qquad R^1 - (OC_nH_{2n})_x - \overset{(+)}{\underset{(C_nH_{2n}O)_zH}{\overset{R^3}{N}}} - (C_nH_{2n}O)_y - R^2 \qquad A^{(-)}$$

in welcher $R^1$ und $R^2$ lineare Alkylgruppen mit 8 bis 22 C-Atomen, $R^3$ eine Alkylgruppe mit 1 bis 4 C-Atomen, eine Benzylgruppe oder eine Gruppe $-(C_nH_{2n}O)_z-H$, n = 2 oder 3, x, y und z Zahlen von 1 bis 20 und A ein Chlorid, Bromid oder ein Anion der Formel $R^4OSO_3^{(-)}$, worin $R^4$ eine Alkylgruppe mit 1 bis 4 C-Atomen ist, darstellen.

2. Mittel nach Anspruch 1 zur Nachbehandlung der Haare, dadurch gekennzeichnet, daß es in Form einer Öl-in-Wasser-Emulsion vorliegt und 0,1 bis 5 Gew.-% der quartären Ammoniumverbindung der Formel 1 und zusätzlich eine wasserunlösliche Fettkomponente aus der Gruppe Fettalkohole mit 12 bis 18 C-Atomen, Fettsäuremono-und/oder -diglyceride von Fettsäuren mit 12 bis 18 C-Atomen oder Paraffine in einer Menge von 0,2 bis 10 Gew.-% enthält.

3. Mittel nach Anspruch 1 zur Pflege der Haare, dadurch gekennzeichnet, daß es in Form einer klaren wäßrigen oder wäßrig-alkoholischen Lösung vorliegt und 0,1 bis 5 Gew.-% der quartären Ammoniumverbindung der Formel 1 und zusätzlich ein wasserlösliches Polymeres in einer Menge von 0,1 bis 5 Gew.-% enthält.

4. Mittel nach den Ansprüchen 1 bis 3, dadurch gekennzeichnet, daß in der Formel 1 $R^1$ und $R^2$ lineare Alkylgruppen mit 16 bis 18 C-Atomen und $R^3$ eine Methylgruppe oder Hydroxyethylgruppe ist.